# EUROPEAN PATENT SPECIFICATION

(11) **EP 2 005 177 B1**
(45) Date of publication and mention of the grant of the patent: **10.07.2013**
(21) Application number: 07727885.1
(22) Date of filing: 06.04.2007
(51) Int. Cl.: G01N 33/542, G01N 33/569

(54) **A HOMOGENEOUS TIME RESOLVED FLUORESCENCE BASED TEST METHOD**
HOMOGENE ZEITAUFGELÖSTE TESTMETHODE AUF FLUORESZENZBASIS
MÉTHODE DE TEST BASÉ SUR LA FLUORESCENCE EN TEMPS RÉSOLU EN MILIEU HOMOGÈNE

(30) Priority: 06.04.2006 EP 06112283
(43) Date of publication of application: 24.12.2008
(73) Proprietor: Janssen R&D Ireland, Eastgate, Little Island, County Cork (IE)
(72) Inventor: DAMS, Géry Karel Julia, 3583 Paal-Beringen (BE); VEREYCKEN, Inge, 2100 Deurne (BE); VAN ACKER, Koenraad Lodewijk August, 9140 Temse (BE); GUSTIN, Emmanuel Marie Paul Ernest, 2350 Vosselaar (BE); VERSCHUEREN, Wim Gaston, 2600 Berchem (BE); OHAGEN, Åsa Catrine, G1 1EJ Glasgow (GB)
(74) Representative: Daelemans, Frank F.R.
(86) International application number: PCT/EP2007/053417
(87) International publication number: WO 2007/113337

(56) References cited:
- WO-A-03/027255
- WO-A-2004/076640
- WO-A-2005/118886
- US-A1- 2002 077 284
- US-A1- 2005 014 169
- CUMMINGS R T ET AL: "Use of a phosphotyrosine-antibody pair as a general detection method in homogeneous time-resolved fluorescence: application to human immunodeficiency viral protease" ANALYTICAL BIOCHEMISTRY, ACADEMIC PRESS, SAN DIEGO, CA, US, vol. 269, 10 April 1999 (1999-04-10), pages 79-93, XP002975213 ISSN: 0003-2697
- SELVIN P R ET AL: "LUMINESCENCE ENERGY TRANSFER USING A TERBIUM CHELATE: IMPROVEMENTS ON FLUORESCENCE ENERGY TRANSFER" PROCEEDINGS OF THE NATIONAL ACADEMY OF SCIENCES OF USA, NATIONAL ACADEMY OF SCIENCE, WASHINGTON, DC, US, vol. 91, October 1994 (1994-10), pages 10024-10028, XP001018013 ISSN: 0027-8424
- KAO R Y T ET AL: "A SMALL-MOLECULE INHIBITOR OF THE RIBONUCLEOLYTIC ACTIVITY OF HUMAN ANGIOGENIN THAT POSSESSES ANTITUMOR ACTIVITY" PROCEEDINGS OF THE NATIONAL ACADEMY OF SCIENCES OF USA, NATIONAL ACADEMY OF SCIENCE, WASHINGTON, DC, US, vol. 99, no. 15, 23 July 2002 (2002-07-23), pages 10066-10071, XP002271723 ISSN: 0027-8424
- JENKINS J L ET AL: "VIRTUAL SCREENING TO ENRICH HIT LISTS FROM HIGH-THROUGHPUT SCREENING: A CASE STUDY ON SMALL-MOLECULE INHIBITORS OF ANGIOGENIN" PROTEINS: STRUCTURE, FUNCTION AND GENETICS, ALAN R. LISS, US, vol. 50, no. 1, 1 January 2003 (2003-01-01), pages 81-93, XP008062395 ISSN: 0887-3585
- JIANG S ET AL: "A SCREENING ASSAY FOR ANTIVIRAL COMPOUNDS TARGETED TO THE HIV-1 GP41 CORE STRUCTURE USING A CONFORMATION-SPECIFIC MONOCLONAL ANTIBODY" JOURNAL OF VIROLOGICAL METHODS, AMSTERDAM, NL, vol. 80, 1999, pages 85-96, XP001013466 ISSN: 0166-0934

### SEQUENCE LISTING

<110> Tibotec Pharmaceuticals Ltd.
<120> A homogeneous time resolved fluorescence based test system
<130> TIP 126 PCT
<150> EP 06112283.4
   <151> 2006-04-06
<160> 5
<170> PatentIn version 3.3
<210> 1
   <211> 66
   <212> PRT
   <213> Human immunodeficiency virus
<400> 1
   Arg Met Lys Gln Ile Glu Asp Lys Ile Glu Glu Ile Glu Ser Lys Gln
   1               5                   10                  15
   Lys Lys Ile Glu Asn Glu Ile Ala Arg Ile Lys Lys Leu Ile Ser Gly
               20                  25                  30
   Ile Val Gln Gln Gln Asn Asn Leu Leu Arg Ala Ile Glu Ala Gln Gln
           35                  40                  45
   His Leu Leu Gln Leu Thr Val Trp Gly Ile Lys Gln Leu Gln Ala Arg
       50                  55                  60
   Ile Leu
   65
<210> 2
   <211> 34
   <212> PRT
   <213> Human immunodeficiency virus
<400> 2
   Trp Met Glu Trp Asp Arg Glu Ile Asn Asn Tyr Thr Ser Leu Ile His
   1               5                   10                  15
   Ser Leu Ile Glu Glu Ser Gln Asn Gln Gln Glu Lys Asn Glu Gln Glu
               20                  25                  30
   Leu Leu
<210> 3
   <211> 45
   <212> PRT
   <213> Human immunodeficiency virus
<400> 3
   Arg Met Lys Gln Ile Glu Asp Lys Ile Glu Glu Ile Glu Ser Lys Gln
   1               5                   10                  15
   Lys Lys Ile Glu Asn Glu Ile Ala Arg Ile Lys Lys Leu Leu Gln Leu
               20                  25                  30
   Thr Val Trp Gly Ile Lys Gln Leu Gln Ala Arg Ile Leu
           35                  40                  45
<210> 4
   <211> 45
   <212> PRT
   <213> respiratory syncytial virus
<400> 4
   Asn Phe Tyr Asp Pro Leu Val Phe Pro Ser Asp Glu Phe Asp Ala Ser
   1               5                   10                  15
   Ile Ser Gln Val Asn Glu Lys Ile Asn Gln Ser Leu Ala Phe Ile Arg
               20                  25                  30
   Lys Ser Asp Glu Leu Leu His Asn Val Asn Ala Gly Lys
           35                  40                  45
<210> 5
   <211> 36
   <212> PRT
   <213> Human immunodeficiency virus
<400> 5
   Tyr Thr Ser Leu Ile His Ser Leu Ile Glu Glu Ser Gln Asn Gln Gln
   1               5                   10                  15
   Glu Lys Asn Glu Gln Glu Leu Leu Glu Leu Asp Lys Trp Ala Ser Leu
               20                  25                  30
   Trp Asn Trp Phe
           35

## Description

Current therapy for the treatment of human immunodeficiency virus (HIV) generally targets the viral enzymes reverse transcriptase and protease. However, several other gene products of HIV, such as the envelope glycoprotein, also play critical roles in infection.

The envelope glycoprotein consists of two associated subunits, gp120 and gyp41, generated by proteolytic cleavage of the precursor gp160 protein. It resides in the viral membrane as a complex of three gp120 and three gp41 subunits. It is the gp41 subunit that mediates fusion of the membranes of the virus and target cell, allowing the HIV to infect new cells. The gp120 subunit is involved in target cell recognition and receptor binding.

The process of membrane fusion mediated by gp41 involves a conformational change in the glycoprotein, exposing in the target cell membrane a trimeric coiled coil formed by alpha helices from the N-terminal region of each of the three gp41 subunits (the N-helix). This coiled coil interacts with alpha helices from the C-terminal region of the three-gp41 subunits (the C-helix). The resulting hexameric alpha helical interaction between the N-helix and the C-helix regions of gp41 fuses the viral and cellular membranes.

Proteolytic studies were used to identify the gp41 segments responsible for formation of the hexameric fusion intermediate, called N36 and C34. (D.C. Chan et al., Cell 89:263-273 (1997). Intriguingly, residues within the N36 and C34 regions are some of the most highly conserved residues of the envelope coding region of the HIV genome. Several mutations that inhibit membrane fusion and abolish infectivity map to these regions. Moreover, nanomolar concentrations of synthetic peptides corresponding to these regions have been shown to inhibit HIV infectivity and syncytia formation in cell culture, suggesting that they act as inhibitors of gp41-mediated membrane fusion. These results indicated that an isolated complex between peptides comprising amino acids sequences from the N36 and C34 regions would be a good model of the gp41 fusogenic intermediate.

Synthetic N36 and C34 peptides were shown to form a stable hexameric structure under appropriate conditions *in vitro,* indicating that peptides containing amino acid sequences from these regions can form the hexameric gp41 core in the absence of the remainder of the gp41 subunit. The X-ray crystal structure of the N36/C34 complex was determined by D.C. Chan et al., Cell 89:263-273 (1997). The structure revealed the three C34 peptides representing the C-helix of gp41 were packing in an antiparallel fashion against the three N36 peptides representing the N-helix, forming a six-helix bundle.

The N-helices form an interior trimeric coiled coil with three hydrophobic grooves. The hydrophobic cavities are filled by three residues of the C-helix, Trp 628, Trp 631, and Ile 635. In contrast, residues of the C-helix such as Met 629, Gln 630, and Arg 633 lie on the outside of the hexamer, and make no contacts with the N-helix.

The N-helix residues that form the hydrophobic pocket (residues 565-577) are highly conserved among HIV strains. The RNA that encodes these residues is also part of the Rev-response element, a highly structured RNA critical for the viral lifecycle.

The C34 peptide has been shown to be a potent inhibitor of HIV infectivity and membrane fusion. Alanine mutagenesis studies on C34 showed that mutation of the residues corresponding to Trp 628, Trp 631, or Ile 635 to alanine had significant effects on both C34 inhibition of membrane fusion and on complex formation with N36. Alanine mutation of either Met 629 or Arg 633, which do not contact the N-helix, had no significant effect on either membrane fusion or on C34/N36 complex formation. (D.C. Chan et al., Proc. Natl. Acad. Sci., U.S.A. 95:15613-7 (1998) .) These data indicated that the hydrophobic interactions involving residues 565-577 of the N-helix and residues Trp 628, Trp 631, and Ile 635 of the C-helix play an important role in stabilizing the hexameric alpha helical bundle for membrane fusion.

This hydrophobic interaction is an attractive target for candidate gp41 inhibitors. While this interaction is present in the N36/C34 peptide complex, this complex is not ideally suited for screening of potential gp41 inhibitors because N36 is insoluble and subject to aggregation in the absence of C34. (M. Lu et al. Nat. Struct. Biol. 2: 1075-1082: D.M. Eckert et al. Cell 99: 104). Therefore, a soluble fusion peptide comprising the C-terminal 17 residues of N36 and 29 residues of GCN4-pI_{Q}I was constructed (with a 1 residue overlap between the two regions, making the peptide 45 residues long). (D.M. Eckert et al. Cell 99: 105.) This peptide, called IQN17, has the sequence RMKQIEDKIEEIESKQKKIENE1ARIKKLLQLTVWGIKQLQARIL (SEQ ID NO: 3), with the HIV-1 gp41 sequence of amino acids 565-581 underlined. IQN17 includes 3 mutations of surface residues in the GCN4-pI_{Q}I region to improve solubility. It is fully helical, with nearly the same superhelix parameters as the gp41 N-helix, and forms a stable trimer in solution. The X-ray crystal structure of IQN17 in complex with a cyclic peptide (D-peptide) showed that the hydrophobic pocket formed by residues 565-577 is nearly identical to that of N36. (WO 00/06599).

International application WO 00/06599 describes the use of IQN17 as a mimic of the N-helix region of gp41 for identification of candidate D-peptide membrane fusion inhibitors. A library of D-peptide molecules designed to present hydrophobic moieties into the binding pocket of IQN17 was tested in screening assays using mirror image phage display. However, to date, the use of IQN17 has been limited to D-peptides. Furthermore WO 00/06599 does not provide a simple assay for identifying anti-fusion compounds.

Inhibition of the formation of a stable six-helix bundle offers an interesting approach to prevent HIV infection. As mentioned above, HIV is surrounded by a lipid bi-layer bearing envelope proteins composed of three heavily glycosylated gp120 proteins on the exterior and three gp41 transmembrane glycoproteins on the interior. The molecular sequence of gp41 includes so-called "heptad-repeat" regions (HR1 and HR2 respectively). A heptad-repeat is a type of tandem repeat sequence in which a group of seven amino acids occurs many times in a protein sequence. Entry of HIV into the host cell begins with the binding of gp120 to the cellular CD4 receptor and its subsequent binding to the chemokine co-receptors CCR5 or CXCR4. This triggers a so-called spring-loaded mechanism that unmasks the gp41 fusion domain, causing it to spring toward the cell membrane. The HR1 regions then fold over into the hydrophobic grooves formed by the corresponding HR2 regions, resulting in a stable 6-helix bundle. This brings viral and cell membranes into proximity for fusion and entry. Hence, interfering with 6-helix bundle formation prevents the virus from entering the cell.

On another aspect, goals of drug design initially comprise the characterization of selectivity and affinity, efficacy, toxicity (therapeutic window/safety margin), pharmacokinetics, and stability, for a given compound. One of the earlier steps in drug design and discovery is focused on discriminating potential active compounds amongst a large variety of chemical compounds. This discrimination of potential active chemical structures is suitably accomplished by ligand-binding assays. Ligand-binding assays measure the affinity and/or degree of a drug to remain associated with a receptor. Within a molecule structure, the affinity or degree of binding of a specific moiety for a target recognition site of the receptor is particularly useful information in designing and optimizing effective compounds against a given target. Additionally, ligand-binding assays are especially convenient in elucidating mechanisms of action or inhibition.

Although screening assays for fusion inhibitors have been available for some time, mechanistic studies of HIV fusion inhibitors targeted to gp41 have been hampered by the lack of sensitive methodologies, which may discriminate between a large range of degrees of binding, leaving a tremendous range of chemical diversity untested.

One of the known screening assays from WO 03/027255 is the so-called capillary zone electrophoresis (CZE) allowing the detection and discrimination of compounds or moieties of compounds useful for designing HIV fusion inhibitors. With CZE the degree of complex formation between two helical peptides can be identified. The endpoint of this technology relies on disruption of the complexing peptides. However, compounds inducing subtle distortions of peptide interactions exhibit equal anti-fusion activity.

A model and the means for directly measuring ligand-binding with high sensitivity and robustness is highly desired for screening a broader window of anti-fusion compounds.

A fluorescence resonance energy transfer based test system detects distortion as well as disruption of the peptide interaction by any tested compound, because angstrom scale distance changes lead to signal changes. In addition the throughput of such a system for compound evaluation is much higher compared to e.g. the CZE technology.

The invention also circumvents the classical problem encountered in fluorescence assays of intrinsic fluorescent properties of many test compounds by using europium. Europium exhibits an exceptionally long-lived fluorescence and thus the energy transfer to allophycocyanin is measured milliseconds after excitation when intrinsic compound fluorescence is less significant.

Many compounds and proteins present in biological fluids or serum are naturally fluorescent, and the use of conventional fluorophores may lead to serious limitations of sensitivity. Most of these background signals being short-lived, the use of long-lived labels combined with detection on a time-resolved fluorescence basis surprisingly allow the minimization of prompt interferences.

In the fluorescence resonance energy transfer technology the dependence of the energy transfer rate is postulated on the inverse sixth power of the distance between an excited fluorescent donor and a nearby acceptor molecule.

The current invention relates to a method for identifying a compound that interferes with the formation of the HIV six-helix bundle of gp41 comprising: providing a first helical polypeptide consisting of the sequence of IQN36 (SEQ ID NO:1);
providing a second helical polypeptide consisting of the sequence of C34 (SEQ ID NO: 2);
wherein said IQN36 is labeled with a light emitting fluorophore and said C34 is labeled with an ultra-violet excitable fluorophore and wherein
the light emitting fluorophore is allophycocyanin, preferably streptavidin-allophycocyanin, and the ultra-violet excitable fluorophore is europium,
providing a test composition comprising the compound;
measuring the degree of complex formation between the first helical polypeptide and the second helical polypeptide in the presence of the test composition comprising the compound using fluorescence resonance energy transfer; and
comparing the measured degree of complex formation to the degree of complex formation between the first helical polypeptide and the second helical polypeptide in the absence of the test composition comprising the compound to identify the compound that interferes with the formation of the HIV six-helix bundle of gp41.

Said IQN36 sequence may comprise a linker between the label and the IQ-moiety of the IQN36 sequence, preferably the linker is attached to the N-terminal IQ-end of the IQN36 sequence.

The linker is selected from the group of an antibody-antibody complex, antibody-antigen complex or streptavidin-biotin system.

The compounds identified by the above-mentioned method are listed in the Example section of the present description and the thus identified compounds can be used for inhibiting the formation of the HIV six-helix bundle of gp41.

These terms as used herein are defined as follows:
Helical polypeptide as used herein refers to a polypeptide with a helical content of at least 70% in aqueous solution, such as for example 74%. 80%, 85%. 90% and 95%. The percent helical content is estimated as previously described (Sreerama et al., Anal. Biochem. 209:32-44 (1993)).

A fusion inhibitor, as used herein, is any compound that prevents membrane fusion between target cells and free virus or viral infected cells. For example, a HIV fusion inhibitor may be any compound that binds to gp41 and prevents the fusogenic six-helical bundle formation, thus decreases gp41-mediated membrane fusion. In one embodiment, a fusion inhibitor is any compound that decreases the degree of complex formation or binding affinity. In another embodiment, a fusion inhibitor is chosen from peptides, derivatized peptides, C-peptides, D-peptides, N-peptides, cyclic or linear, small and large molecules that decrease gp41-mediated membrane fusion, including, for example, disrupting the complex formation of the N- and C-helices of gp41.
C-peptides are peptide segments derived from the second heptad repeat region of HIV gp41 sequence and their derivatives, including C34, C28, T20, and T1249.

N-peptides are peptide segments derived from the first heptad repeat region of HIV gp41 sequence and their derivatives, including N36 and DP107.

A test composition comprises any compound, including, but not limited to, peptides, dipeptides, tripeptides, polypeptides, proteins, small and large organic molecules and derivatives thereof. Large organic molecules are those with a molecular weight higher than 1000 Daltons.

Complex formation or binding affinity, as used herein, refers to the ability of at least two entities, for example, at least two peptides, to interact with one another, such as, for example, by hydrogen bonding and Van der Waals interactions. The degree of complex formation of two peptides would therefore be the extent of interaction between two peptides. This parameter ranges between 0-100%, with 100% being one peptide completely bound to the other peptide at the experimental concentrations.

The binding affinity of the first helical polypeptide and the second helical polypeptide, both alone and in the presence of the test composition, may be measured by any method known in the art. For example, the binding affinity may be measured by titrating the second helical polypeptide against a fixed concentration of the first helical polypeptide or vice versa.

The degree of complex formation measures the percentage of bound second helical polypeptide relative to the total amount of second helical polypeptide, at fixed concentrations of the first and second helical polypeptides. Although one can calculate binding affinity from the degree of complex formation, this is usually not recommended because of possible large errors. The difference between degree of complex formation and binding affinity is that binding affinity is usually determined by a series of measurements of degree of complex formation at a fixed concentration of one binding component, and at increasing concentrations of the second binding component until the first binding component is completely bound. A titration curve is thus obtained.

Peptides mentioned or used in the current description are:
SEQ ID NO 1 (HIV- IQN36)
SEQ ID NO 2 (HIV- C34):
   WMEWDREINNYTSLIHSLIEESQNQQEKNEQELL
SEQ ID NO 3 (HIV- IQN17):
   RMKQIEDKIEEIESKQKKIENEIARIKKLLQLTVWGIKQLQARIL
SEQ ID NO 4: (RSV- C45):
   NFYDPLVFPSDEFDASISQVNEKINQSLAFIRKSDELLHNVNAGK
SEO ID NO 5 (T20):
   YTSLIHSLIEESQNQQEKNEQELLELDKWASLWNWF

### Examples

Streptavidin-allophycocyanin (S-APC) was purchased from Perkin Elmer and peptides C34, T20, C45, biotin-labeled IQN36 (IQN36-biotin) and europium-labeled C34 (C34-eu) were synthesized according to standard protocols. All reagents were dissolved in 100 mM Hepes buffer pH 7.2. Test compounds were serially diluted and then added to 384-well plates. A final concentration of 10 nM S-APC and 100 nM IQN36-biotin were mixed in tubes, incubated for 30 min at room temperature and then added to well containing test compound. The compound/S-APC/IQN36-biotin mixture was incubated for another 2 hours at room temperature. After the incubation, C34-eu was added at a final concentration of 125 nM followed by a final incubation of the mixture for 30 min at room temperature. After the incubation, the fluorescence resonance energy transfer (FRET) signal was detected using a Viewlux reader and used to calculate the 50% inhibitory concentration (IC₅₀) of the test compound.
IC₅₀ is the drug concentration at which 50% of the FRET signal (or 6HB complex formation) is inhibited.

### Alternative assays set-ups

The assays principle can be applied to a number of different capture techniques.
Instead of the streptavidin-biotin system for peptide capture to the assay plate, antibodies targeting the peptides can be used.

### Results

Two peptides with reported inhibitory activity against 6-helix bundle (HB) formation (T20 and C34) and an irrelevant negative control peptide derived from the RSV F-protein (C45) were used to validate the method. T20 and C34 showed inhibition of the 6-HB formation and FRET signal whereas C45 showed no inhibitory activity up to 10 µM.

| **Peptide** | **IC₅₀ (µM)** |
|---|---|
| T20 | 1 ± 0.5 |
| C34 | 0.07 ± 0.04 |
| C45 | > 10 |

The well-established fusion inhibitors C34 and T20 exhibit potent activity in this test system. Diverse compound libraries in a high-throughput screening campaign were evaluated in order to identify several inhibitors falling into different chemical classes with low micromolar IC₅₀. Among those were compounds that proved to be active in a cell-based antiviral assay, in the absence of cytotoxicity. (Table 1)

Validation of the assay was done with various unlabeled HR2-peptides and small molecules with putative 6-helix bundle inhibitory activity. Different FRET couples were tested, quenching issues were addressed, and the assay was optimized for high-throughput screening (HTS). Under optimal conditions, the assay proved to have a convenient dynamic range and low signal measurement-associated data variation (z-factor = 0.8, signal/noise > 10). It was found to be selective towards HIV-1 fusion inhibitors, and when comparing several parallel experiments, data were reproducible. Evaluation of diverse in-house libraries in a HTS campaign, allowed identifying different chemical scaffolds that interact with this molecular mechanism.

**Table 1**

| Compound No | Structure | HIV-FRET-IQN36_C34 IC₅₀ (µM) |
|---|---|---|
| 1 | 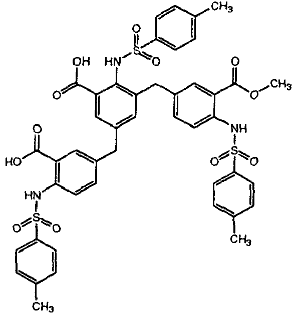 | 3.27 |
| 2 | 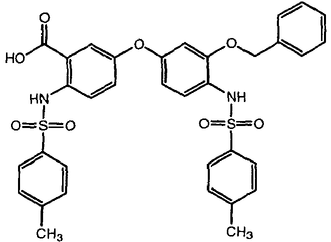 | 2.72 |
| 3 | 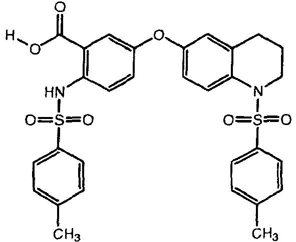 | 2.88 |
| 4 | 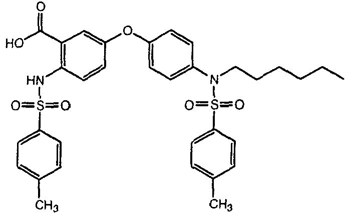 | 4.45 |
| 5 | 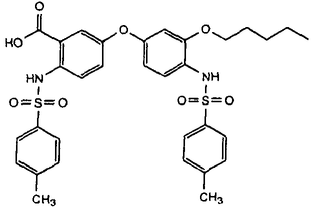 | 3.48 |
| 6 | 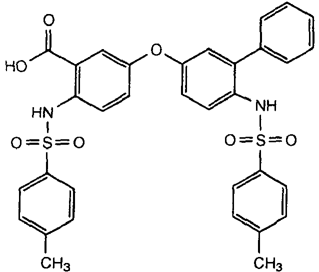 | 3.16 |
| 7 | 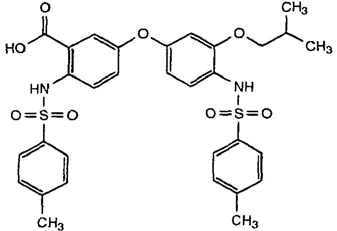 | 3.27 |
| 8 | 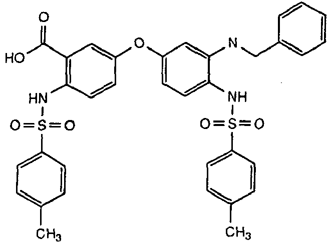 | 2.27 |
| 9 | 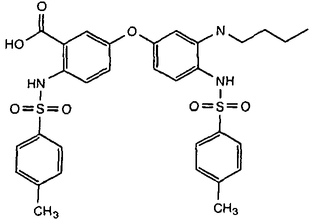 | 1.81 |
| 10 | 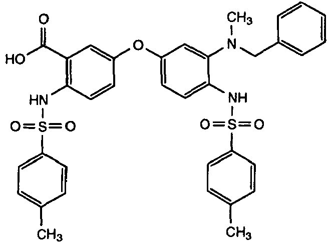 | 2.64 |
| 11 | 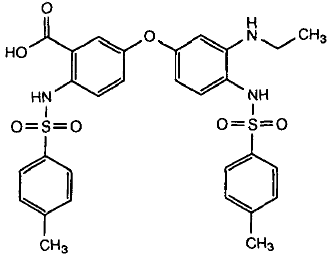 | 2.47 |
| 12 | 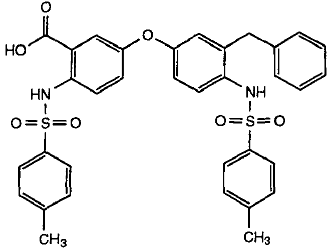 | 2.09 |
| 13 | 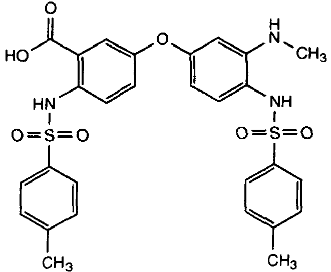 | 2.69 |

## Claims

1. Method for identifying a compound that interferes with the formation of the HIV six-helix bundle of gp41 comprising:
providing a first helical polypeptide consisting of the sequence of IQN36 (SEQ ID NO:1);
providing a second helical polypeptide consisting of the sequence of C34 (SEQ ID NO: 2) wherein said IQN36 is labeled with a light emitting fluorophore and said C34 is labeled with an ultra-violet excitable fluorophore; and wherein the light emitting fluorophore is allophycocyanin, preferably streptavidin-allophycocyanin, and the ultra-violet excitable fluorophore is europium.
providing a test composition comprising the compound;
measuring the degree of complex formation between the first helical polypeptide and the second helical polypeptide in the presence of the test composition comprising the compound using fluorescence resonance energy transfer; and
comparing the measured degree of complex formation to the degree of complex formation between the first helical polypeptide and the second helical polypeptide in the absence of the test composition comprising the compound to identify the compound that interferes with the formation of the HIV six-helix bundle of gp41.

2. Method for identifying a compound that interferes with the formation of the HIV six-helix bundle of gp41 according to claim 1 wherein said IQN36 sequence comprises a linker between the label and the IQ-moiety of the IQN36 sequence.

3. Method for identifying a compound that interferes with the formation of the HIV six-helix bundle of gp41 according to claim 2 wherein the linker is attached to the N-terminal IQ-end of the IQN36 sequence.

4. Method for identifying a compound that interferes with the formation of the HIV six-helix bundle of gp41 according to claim 2 or 3 wherein said linker is selected from the group of an antibody-antibody complex, antibody-antigen complex or streptavidin-biotin system.

## Patentansprüche

1. Verfahren zum Identifizieren einer Verbindung, die die Bildung des sechs-Helix-Bündels von gp41 von HIV stört, umfassend:
Bereitstellen eines ersten helikalen Polypeptids, das aus der Sequenz von IQN36 (SEQ ID NO: 1) besteht;
Bereitstellen eines zweiten helikalen Polypeptids, das aus der Sequenz von C34 (SEQ ID NO: 2) besteht, wobei das IQN36 mit einem lichtemittierenden Fluorophor markiert ist und das C34 mit einem Ultraviolett-anregbaren Fluorophor markiert ist; und wobei das lichtemittierende Fluorophor Allophycocyanin ist, vorzugsweise Streptavidin-Allophycocyanin, und das Ultraviolett-anregbare Fluorophor Europium ist;
Bereitstellen einer Prüfzusammensetzung, die die Verbindung umfasst;
Messen des Grads an Komplexbildung zwischen dem ersten helikalen Polypeptid und dem zweiten helikalen Polypeptid in Gegenwart der Prüfzusammensetzung, die die Verbindung umfasst, unter Verwendung von Fluoreszenzresonanzenergietransfer; und Vergleichen des gemessenen Grads an Komplexbildung mit dem Grad an Komplexbildung zwischen dem ersten helikalen Polypeptid und dem zweiten helikalen Polypeptid bei Abwesenheit der Prüfzusammensetzung, die die Verbindung umfasst, um die Verbindung zu identifizieren, die die Bildung des sechs-Helix-Bündels von gp41 von HIV stört.

2. Verfahren zum Identifizieren einer Verbindung, die die Bildung des sechs-Helix-Bündels von gp41 von HIV stört, gemäß Anspruch 1, wobei die IQN36-Sequenz einen Linker zwischen der Markierung und der IQ-Einheit der IQN36-Sequenz umfasst.

3. Verfahren zum Identifizieren einer Verbindung, die die Bildung des sechs-Helix-Bündels von gp41 von HIV stört, gemäß Anspruch 2, wobei der Linker an das N-terminale IQ-Ende der IQN36-Sequenz gebunden ist.

4. Verfahren zum Identifizieren einer Verbindung, die die Bildung des sechs-Helix-Bündels von gp41 von HIV stört, gemäß Anspruch 2 oder 3, wobei der Linker ausgewählt ist aus der Gruppe von einem Antikörper-Antikörper-Komplex, Antikörper-Antigen-Komplex und einem Streptavidin-Biotin-System.

## Revendications

1. Méthode d'identification d'un composé qui interfère avec la formation du faisceau à six hélices de gp41 de VIH comprenant :
l'obtention d'un premier polypeptide en hélice constitué de la séquence de IQN36 (SEQ ID NO:1) ;
l'obtention d'un deuxième polypeptide en hélice constitué de la séquence de C34 (SEQ ID NO:2), ledit IQN36 étant marqué par un fluorophore photoémetteur et ledit C34 étant marqué par un fluorophore excitable par les ultra-violets, et le fluorophore photoémetteur étant l'allophycocyanine, de préférence la streptavidine-allophycocyanine, et le fluorophore excitable par les ultra-violets étant l'europium ;
l'obtention d'une composition à tester comprenant le composé ;
la mesure du degré de formation de complexe entre le premier polypeptide en hélice et le deuxième polypeptide en hélice en présence de la composition à tester comprenant le composé en utilisant le transfert d'énergie par résonance de fluorescence ; et
la comparaison du degré de formation de complexe mesuré avec le degré de formation de complexe entre le premier polypeptide en hélice et le deuxième polypeptide en hélice en l'absence de la composition à tester comprenant le composé pour identifier le composé qui interfère avec la formation du faisceau à six hélices de gp41 de VIH.

2. Méthode d'identification d'un composé qui interfère avec la formation du faisceau à six hélices de gp41 de VIH selon la revendication 1, **caractérisée en ce que** ladite séquence IQN36 comprend un lieur entre le marqueur et le motif IQ de la séquence IQN36.

3. Méthode d'identification d'un composé qui interfère avec la formation du faisceau à six hélices de gp41 de VIH selon la revendication 2, **caractérisée en ce que** le lieur est lié à l'extrémité N-terminale IQ de la séquence IQN36.

4. Méthode d'identification d'un composé qui interfère avec la formation du faisceau à six hélices de gp41 de VIH selon la revendication 2 ou 3, **caractérisée en ce que** ledit lieur est choisi dans le groupe constitué par un complexe anticorps-anticorps, un complexe anticorps-antigène ou un système streptavidine-biotine.
